(19) Europäisches Patentamt
European Patent Office
Office européen des brevets

(11) **EP 2 412 869 B1**

(12) **EUROPÄISCHE PATENTSCHRIFT**

(45) Veröffentlichungstag und Bekanntmachung des Hinweises auf die Patenterteilung:
**06.03.2013 Patentblatt 2013/10**

(51) Int Cl.:
***D21G 9/00*** *(2006.01)*

(21) Anmeldenummer: **10171493.9**

(22) Anmeldetag: **30.07.2010**

(54) **Verfahren zur Herstellung von bahnförmigen Material**

Method for producing sheet-like material

Procédé de fabrication de matériau en forme de bande

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**BA ME RS**

(43) Veröffentlichungstag der Anmeldung:
**01.02.2012 Patentblatt 2012/05**

(73) Patentinhaber: **Mitsubishi HiTec Paper Europe GmbH**
**33699 Bielefeld (DE)**

(72) Erfinder:
• **Bachmann, Bernhard, Prof. Dr.**
 **33739 Bielefeld (DE)**
• **Ueckerdt, Rainer, Prof. Dr. Dr.**
 **12587 Berlin (DE)**
• **Penner, Hermann**
 **33605 Bielefeld (DE)**
• **Becker, Dieter, Dr.**
 **49124 Georgsmarienhütte (DE)**
• **Rodewald, Stefan**
 **33719 Bielefeld (DE)**
• **Elsner, Christian**
 **33818 Leopoldshöhe (DE)**

(56) Entgegenhaltungen:
**EP-A1- 1 473 407      WO-A1-03/072874**
**WO-A1-2010/080869**

EP 2 412 869 B1

## Beschreibung

Die Erfindung betrifft ein Verfahren zur Herstellung und/oder Verarbeitung eines bahnförmigen Materials, insbesondere von Papier, mit mindestens einem bevorzugt traversierenden Messsystem, das innerhalb eines festen Zeitintervalls At über die Bahnbreite s des bahnförmigen Materials an n verschiedenen Messpositionen pro Messposition jeweils m Messwerte aufnimmt.

Solche Messsysteme sind insbesondere in der Papier herstellenden Industrie seit vielen Jahren bekannt und dienen dort der laufenden Prozess- und Qualitätskontrolle.

Problematisch an den bisher bekannten Systemen ist die Tatsache, dass die bekannten Messsysteme im Endeffekte eine riesige Zahl einzelner Messwerte oder Messprofile als grafische Darstellung einer Vielzahl aufgenommener Messwerte generieren, die anschließend einer subjektiven Deutung gewöhnlich durch die Produktionsverantwortlichen unterworfen werden. Dabei kommt den Messprofilen entweder als Darstellung von hintereinander aufgenommenen Messwerten an der gleichen Messposition (Längsprofil) oder als Darstellung von Messwerten benachbarter Messpositionen quer über die Bahn des bahnförmig hergestellten Materials (Querprofil) besondere Bedeutung zu, da hier mit großem Engagement nach Trends innerhalb der Produktion gesucht werden kann.

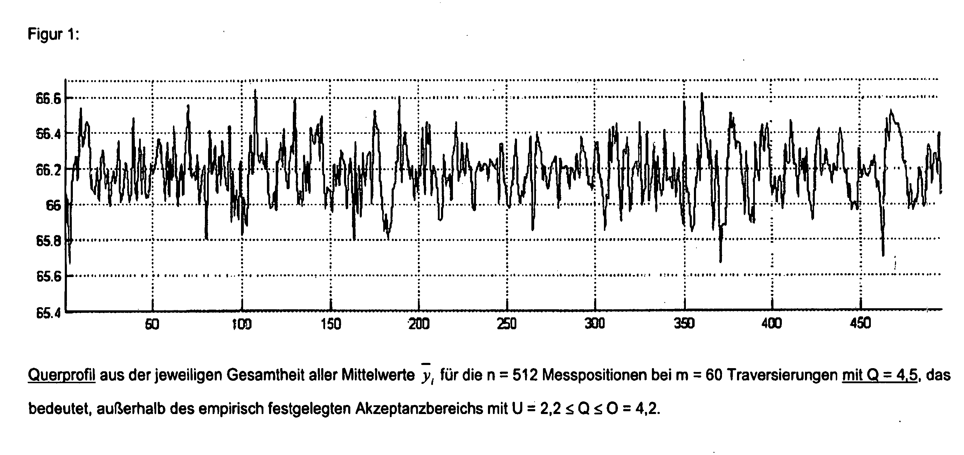

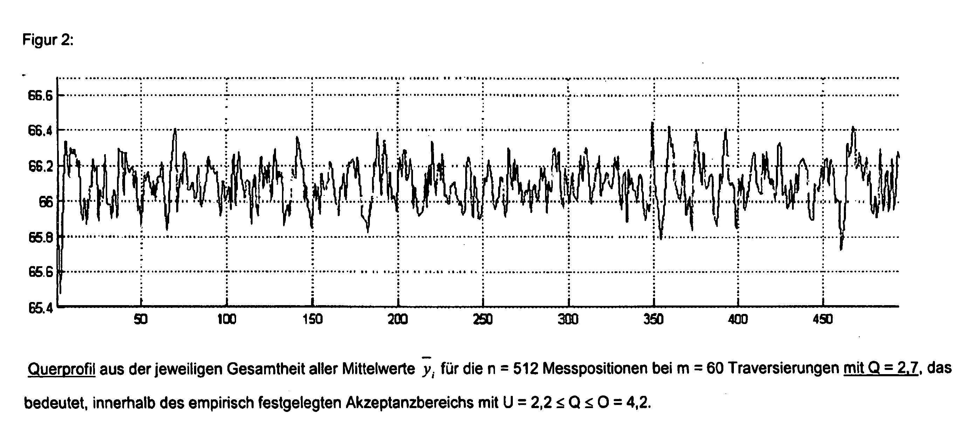

In genau diesem Sinne schlägt die EP 1 473 407 A1 ein Verfahren zur Generierung 2-dimensionaler Grafiken - vgl. dort die Figuren 1 und 2 - von zuvor erzeugten Bahnen aus einem flächigen Material wie Papier oder Folie vor, wobei jene Grafiken zur Darstellung von Längs- und Querprofilen verschiedener Messparameter auf der einen Seite zwar einen sehr hohen Informationsgrad aufweisen, auf der anderen Seite aber gerade deswegen Gegenstand mannigfaltiger Interpretationsmöglichkeiten darstellen. Auch der zweite Ansatz jener Schrift, durch eine Vielzahl an Querprofilen überlappende und wohl auch zyklische Ereignisse innerhalb der Produktion aufzuzeigen, zielt in genau die gleiche Richtung: es wird eine Vielzahl an Zahlenwerten generiert, die grafisch idealerweise 2-dimensional dargestellt werden und die anschließend zu interpretieren sind, was große individuelle Erfahrungen voraussetzt.

Gegenstand der etwas älteren WO 03 / 072 874 A1 ist letztendlich ein Steuergerät zur Optimierung der Faserorientierung innerhalb der Papierproduktion, wozu dem Steuergerät die zuvor aufgenommenen Messwerte der Faserorientierung in Bezug auf Maschinenlauf- und Maschinenquerrichtung der Papierbahn zugeführt werden. Das Steuergerät soll dann durch Eingriff in die Maschinensollwerte die Faserorientierung für die zu produzierende Papierbahn unter Nutzung der bekannten Fuzzy-Logik optimieren. Die Schaffung von Qualitätszahlen als Entscheidungsgrundlage zur Bestimmung gebrauchsgeeigneter/gebrauchsungeeigneter Papiere ist nicht Ziel jener bekannten Schrift.

Schließlich schlägt die WO 2010 / 080 869 A1 ein Verfahren zur Schaffung eines Wahrscheinlichkeitsmodells vor, in dem aus einer Vielzahl von aufgenommenen Messwertprofilen für zuvor bestimmte und eingestellte Maschinenparameter Wahrscheinlichkeitsprofile berechnet werden, um anhand der dann vorliegenden Wahrscheinlichkeitsprofile Entscheidungen für das Maschinenpersonal zur optimalen Einstellung der Maschinensollwerte zu erleichtern. Auch diese Schrift zielt in keinster Weise auf die Schaffung von Qualitätszahlen als einfache Entscheidungsgrundlage zur Bestimmung gebrauchsgeeigneter/gebrauchsungeeigneter Papiere.

Zusammengefasst sind die as dem zuvor gewürdigten Stand der Technik bekannten Verfahren bisher geeignet, aus Messprofilen gewonnene Daten direkt innerhalb des internen Produktionsprozesses zu nutzen oder aber vielmehr, sie gerne insbesondere in komplizierten 2-dimensionalen Grafiken darzustellen. Die so gewonnenen Informationen sind mannigfaltig, ihre Interpretation und Deutung aber äußerst schwierig. Doch selbst nach vielen Jahren Diensterfahrung von mit der Deutung aufgenommener Messprofile beauftragten Produktionsverantwortlichen bleibt das Ergebnis dieser Deutung oft spekulativ und stets höchst subjektiv, ist damit stimmungs- und/oder personenabhängig und letztendlich auch nicht reproduzierbar. Insbesondere bleibt es ein Problem, dass eine einheitliche Produktionssteuerung auf Basis subjektiv gedeuteter Messprofile von dem individuellen Deutungsverhalten einzelner Personen abhängig ist, bei denen die Gefahr eines Weggangs vom Unternehmen immer gegeben ist.

Eine solche Personenabhängigkeit ist für ein Unternehmen stets mit großen Risiken behaftet. Auch bleibt es unbefriedigend, eine industrielle Produktion auf die Basis subjektiv und unter Zeitdruck gedeuteter Messprofile zu stellen.

Es ist also zugrundeliegende Aufgabe für die hier vorliegende Erfindung, ein Verfahren zur Herstellung eines bahnförmigen Materials vorzustellen, das innerhalb des laufenden Prozesses ausschließlich von nachvollziehbaren, im Vorfeld und unter Ausschluss von Zeitdruck bestimmten Parametern bestimmt und gelenkt wird und das infolgedessen nicht auf Basis von spekulativ und subjektiv gedeuteter Messprofile von dem individuellen Deutungsverhalten einzelner Personen abhängig ist.

Die Lösung dieser Aufgabe geschieht mittels eines Verfahrens zur Herstellung und/oder Verarbeitung eines bahnförmigen Materials, insbesondere von Papier, mit mindestens einem Messsystem, das innerhalb eines festen Zeitintervalls $\Delta t$ über mindestens einen Teil der Bahnbreite s des bahnförmigen Materials an n verschiedenen Messpositionen $x_{i\ (i\ =\ 1,..,\ n)}$ pro Messposition jeweils m Messwerte $y_{ik\ (i=1,...,\ n;\ k\ =\ 1,\cdots,\ m)}$ aufnimmt,

wobei das Verfahren dadurch gekennzeichnet ist, dass

das Verfahren die Bestimmung einer rationalen Qualitätszahl Q umfasst, deren Wert für eine als akzeptabel angesehene Qualität des bahnförmigen Materials innerhalb eines für den Herstellungsprozess jeweils empirisch festzulegenden Akzeptanzbereichs mit einer unteren Grenze U und einer oberen Grenze O liegt,
wobei U und O jeweils rationale Zahlen sind mit der Bedingung gemäß:

### Formel 1:

$$U \leq Q \leq O,$$

wobei sich der Wert der Qualitätszahl Q als Funktion von Momenten $M_i$ berechnet gemäß Formel 2 mit:

### Formel 2:

$$Q = f ( M_i )$$

und wobei sich die Momente $M_i$ berechnen aus dem Funktionswert der kubischen Spline-Funktion S(x) für die n verschiedenen Messpositionen gemäß Formel 3 mit:

### Formel 3:

$$S(x) = \alpha_i + \beta_i \cdot (x - x_i) + \gamma_i \cdot (x - x_i)^2 + \delta_i \cdot (x - x_i)^3$$

mit:

x ist Laufvariable in Richtung der Bahnbreite s des bahnförmigen Materials im Intervall $[x_i, x_{i+1}]$
$X_{i\ (i=1,\ldots,\ n)}$ sind die Messpositionen, mathematisch auch als Stützstellen der kubischen Spline-Funktion bezeichnet, für die Momente $M_i$ ,

und wobei für die Momente $M_i$, ausgehend von den zugrundeliegenden Messwerten $y_{ik}$ und unter Nutzung vorhergehender Formel 3 als Darstellung des Funktionswertes der kubischen Spline-Funktion S(x), die Formel 4 gilt mit:

### Formel 4:

$$M_i = 2 \cdot \gamma_{i\ (i = 1,\ldots,\ n-1)}, M_n = 2 \cdot \gamma_{n-1} + \delta_{n-1} \cdot (x_n - x_{n-1})$$

Bevorzugt für das hier vorgeschlagene Verfahren ist ein Messsystem, dass traversierend ausgebildet ist. Dabei ist das Wort Traversierung im Sinne der vorliegenden Erfindung so zu verstehen, dass

- entweder ein Messsystem quer über mindestens einen Teil der Bahnbreite s des bahnförmigen Materials hin und her bewegt wird, so dass nach und nach bei Erreichen der einzelnen n verschiedenen Messpositionen die m verschiedenen Einzelmesswerte $y_{ik}$ aufgenommen werden,

- oder dass ein Messsystem über n verschiedene Messköpfe verfügt, die der Reihe nach abgefragt werden, bis nacheinander alle m Messwerte $y_{ik}$ für alle n verschiedenen Messpositionen aufgenommen worden sind,

- oder dass eine Kombination der beiden vorgenannten Messwertaufnahmen benutzt wird.

Die Umsetzung des hier vorgeschlagenen Verfahrens sieht zunächst die Aufnahme von Messwerten $y_{ik}$ innerhalb eines festen Zeitintervalls $\Delta t$ über mindestens einen Teil der Bahnbreite s des bahnförmigen Materials an n verschiedenen Messpositionen vor, wobei pro Messposition jeweils m Messwerte $y_{ik\ (i=1,\ldots,\ n;\ k=1,\ldots,\ m)}$ aufgenommen werden. Da die einzelnen Messwerte $y_{ik}$ mittels eines bevorzugt traversierenden Messsystems aufgenommen werden, bedeutet das, dass pro einer Traversierung von jeder einzelnen der n verschiedenen Messpositionen jeweils 1 Messwert $y_{ik}$ aufgenommen wird: diese jeweils einmal aufgenommenen Messwerte $y_{ik}$ bilden zusammen dann ein Querprofil über mindestens einen Teil der Bahnbreite s des bahnförmigen Materials. Bei insgesamt m Traversierungen werden so die Messwerte für m Querprofile aufgenommen.
Wie auch immer die Messwerte $y_{ik}$ mittels des bevorzugt traversierenden Messsystems aufgenommen wurden, schließlich liegen für alle n Messpositionen m einzelne Messwerte $y_{ik}$ vor.

Bevorzugt sieht das hier vorgeschlagene Verfahren nunmehr die Bildung des jeweiligen arithmetischen Mittelwertes gemäß Formel 5 vor, mit:

**Formel 5:**

$$\overline{y_i} = \sum_{k=1}^{m} \frac{y_{ik}}{m} \; ,$$

wobei dieser arithmetische Mittelwert für alle n Messpositionen aus den zugehörigen m einzelnen Messwerten $y_{ik}$ jeweils zu bilden ist, so dass schließlich für jede einzelne Messposition n genau ein Mittelwert $\overline{y_i}$ vorliegt.

Die Gesamtheit aller Mittelwerte $\overline{y_i}$ für alle n Messpositionen bilden dann zusammen genau ein Querprofil über mindestens einen Teil der Bahnbreite s des bahnförmigen Materials. Für dieses genau eine Querprofil wird nunmehr bevorzugt mittels mathematischer Berechungsprogramme automatisch die aus n Teilfunktionen bestehende, passende kubische Spline-Funktion für alle n Messpositionen ermittelt gemäß Formel 3 mit:

**Formel 3:**

$$S(x) = \alpha_i + \beta_i \cdot (x - x_i) + \gamma_i \cdot (x - x_i)^2 + \delta_i \cdot (x - x_i)^3 \; \text{ im Intervall } [x_i, x_{i+1}]$$

innerhalb der:

    x ist Laufvariable in Richtung der Bahnbreite s des bahnförmigen Materials im Intervall $[x_i, x_{i+1}]$
    $x_{i\,(i=1..,n)}$ sind die Messpositionen, mathematisch auch als Stützstellen der kubischen Spline-Funktion bezeichnet, für die Momente $M_i$.

S(x) ist gemäß der Formel 3 Funktionswert der kubischen Spline-Funktion, wobei dieser Funktionswert S(x) in den Messpositionen $x_{i\,(i=1,..,n)}$ jeweils durch den arithmetischen Mittelwert der zugehörigen m einzelnen Messwerte für diese Messposition $\overline{y_i} = \sum_{k=1}^{m} \frac{y_{ik}}{m}$ gemäß Formel 5 vorgegeben ist. Das Ergebnis des mathematischen Berechungsprogramms zur Bestimmung der aus n Teilfunktionen bestehenden, passenden kubischen Spline-Funktion ist somit die Bestimmung aller Variablen $\alpha_{i\,(i=1,...,n)}$, $\beta_{i\,(i=1,..,n)}$, $y_{i\,(i=1,..,n)}$ und $\delta_{i\,(i=1,..,n)}$.

Zur Bestimmung des Kurvenverhaltens muss nunmehr für die gesamte kubische Spline-Funktion die zweite Ableitung gebildet werden. Die zweiten Ableitungen pro Intervall sind jeweils gegeben durch Formel 6 mit:

**Formel 6:**

$$S''(x) = 2 \cdot \gamma_i + 6 \cdot \delta_i \cdot (x - x_i) \; \text{ im Intervall } [x_{i-1}, x_i]$$

Innerhalb der Stützstellen für die kubische Spline-Funktion, das sind erfindungsgemäß die Messpositionen, gilt aber $x = x_i$. Damit wird für alle Messpositionen innerhalb von Formel 6 die Differenzbildung $(x - x_i)$ gleich Null.

Angewandt auf Formel 6 ergibt sich an den Stützstellen, das sind erfindungsgemäß die n Messpositionen - siehe oben, für die zweiten Ableitungen Formel 7 mit:

**Formel 7:**

$$S''(x_i) = 2 \cdot \gamma_{i\,(i=1,...,n-1)}, \; S''(x_n) = 2 \cdot \gamma_{n-1} + \delta_{n-1} \cdot (x_n - x_{n-1})$$

Bei den zweiten Ableitungen gemäß Formel 7 spricht man allgemein, und so auch innerhalb dieser Schrift, von

den Momenten ($M_i$) der kubischen Spline-Funktion in den n Messpositionen. Zusätzlich gilt definitionsgemäß für so genannte "natürliche" kubische Spline-Funktionen, dass $M_1 = M_n = 0$ ist. Die Verwendung von natürlichen kubischen Spline-Funktionen innerhalb des erfindungsgemäßen Verfahrens zur Bestimmung einer rationalen Qualitätszahl Q gilt dabei als ganz besonders bevorzugt.

Im Ergebnis der bisherigen Rechnungen liegen also schließlich n Momente ($M_i$) vor, die allesamt der Formel 4 bzw. der Formel 7 genügen. Erfindungsgemäß ist dann gemäß Formel 2 der Wert der Qualitätszahl Q eine Funktion dieser Momente ($M_i$), was bedeutet, dass sich die Qualitätszahl Q aus den n Momente ($M_i$) berechnet.

Zur Berechnung der Qualitätszahl Q aus den Momenten ($M_i$) bieten sich verschiedene Funktionen an, wobei folgende Funktionen als bevorzugt gelten:

- Maximumwert der Absolutwerte (Maximumsnorm) aller Momente ($M_i$),

- Wurzel aus der Summe der Quadrate der Momente $M_i$ (Euklidische Norm), gegeben durch die Formel 8 mit:

### Formel 8:

$$Q = \sqrt{\sum_{i=1}^{n} |M_i|^2}$$

- Varianz aus den Momenten ($M_i$),

- $\sigma$-Regeln beliebiger Stufe aus den Momente $M_i$ ($\sigma$-Wert, 2-$\sigma$-Wert, 3-$\sigma$-Wert, ...),

- Mittelwert aus den Momenten ($M_i$).

Ganz besonders bevorzugt im Sinne der vorliegenden Erfindung ist es, wenn zur Berechnung der Qualitätszahl Q aus den Momenten ($M_i$) als Funktion die Summe der Absolutwerte der Momente ($M_i$) benutzt wird (Betragssummennorm), wenn also zur Berechnung der Qualitätszahl Q aus den Momenten ($M_i$) die Formel 9 gilt, mit:

### Formel 9:

$$Q = \sum_{i=1}^{n} |M_i|$$

Um eine Endkoppelung der Qualitätszahl Q von den Absolutzahlen der Messwerte zu gewährleisten, ist es besonders bevorzugt, wenn die Werte, die sich unmittelbar entsprechend der obigen Vorschläge zur Berechnung der Qualitätszahl Q aus den Momenten (Mi) ergeben, durch den Mittelwert $\sum_{i=1}^{n} \dfrac{\overline{y_i}}{n}$ geteilt werden.

Demnach ist es ganz besonders bevorzugt, wenn die Funktion zur Berechnung der Qualitätszahl Q aus den Momenten $M_i$ die Summe der Absolutwerte der Momente $M_i$ ist, geteilt durch den Mittelwert $\sum_{i=1}^{n} \dfrac{\overline{y_i}}{n}$, gemäß Formel 10, mit:

### Formel 10:

$$Q = \frac{\sum_{i=1}^{n}|M_i|}{\sum_{i=1}^{n}\frac{\bar{y}_i}{n}}$$

Das hier vorgeschlagene Verfahren zur Herstellung und/oder Verarbeitung eines bahnförmigen Materials, insbesondere von Papier, mit dem mindestens einen bevorzugt traversierenden Messsystem umfasst ganz besonders bevorzugt einen zusätz**lichen** Veredelungsprozess, der verändert und/oder unterbunden bzw. gestoppt wird, wenn die Qualitätszahl Q für das zur Veredelung vorgesehene bahnförmige Material außerhalb des Akzeptanzbereichs mit seiner unteren Grenze U und seiner oberen Grenze O liegt. Zu diesem Zweck wird das mindestens eine bevorzugt traversierende Messsystem zur Erfassung der erfindungsgemäß notwendigen Messwerte $y_{ik(i=1,...,n;\ k=1,...,m)}$ so in den Herstellungsprozess integriert, dass die Qualitätszahl Q bestimmt wird, bevor das bahnförmige Material innerhalb des zusätzlichen Veredelungsprozesses behandelt wird. Durch einen manuellen oder bevorzugt automatischen Vergleich dieser Qualitätszahl Q mit den für den Herstellungsprozess empirisch festgelegten Werten zum einen für die untere Grenze U und zum anderen für die obere Grenze O kann dann der Herstellungsprozess verändert und/oder unterbunden bzw. gestoppt werden.

Die Festlegung des Akzeptanzbereichs mit seiner unteren Grenze U und seiner oberen Grenze O, gegebenenfalls mit zusätzlich festgelegten Überwachungsgrenzen $U_1$, $O_1$ und/oder Sonderverwertungsgrenzen $U_2$, $O_2$, bei deren Überschreitung das derart hergestellte bahnförmige Material besonders überwacht oder nur zur eingeschränkten Verwertung gekennzeichnet oder abgezweigt wird, kann vor Aufnahme des normalen Herstellungsprozesses und/oder permanent, jedoch stets außerhalb der Hektik sofort notwendiger Entscheidungen geschehen, beispielsweise im Rahmen von Markt- und aufwendigen Produktionsuntersuchungen:

• So werden zunächst Testproduktionen durchlaufen unter Bestimmung der jeweiligen Qualitätszahl Q. Die fertig gestellten Produkte aus diesen Testproduktionen können anschließend mit beliebigen weiteren Messungen untersucht und die so gewonnenen Messwerte katalogisiert werden.

• Gleichzeitig können die fertig gestellten Produkte aus den Testproduktionen möglichen Kunden vorgestellt werden, um deren Einschätzung der Produkte zu erfahren. Auch ihre jeweilige Einschätzung wird zweckmäßigerweise katalogisiert.

• Anhand der so gewonnenen Messwerte und der Kundeneinschätzungen werden dann die Grenzwerte U und O für den Akzeptanzbereich, gegebenenfalls zusätzlich die Werte $U_1$, $O_1$, $U_2$ und $O_2$ festgesetzt, die dann für die spätere laufende Normalproduktion als Entscheidungswerte für Akzeptanz, Überwachung und/oder Sonderverwertung benutzt werden.

Es ist offensichtlich, das insbesondere der eine, gewöhnlich der obere Grenzwert O bzw. die zusätzlichen oberen Werte $O_1$ und $O_2$ als kritische Werte hinsichtlich schlechter Qualität zur Produktionsunterbrechung bzw. Hinweis auf sofortigen Produktionseingriff genutzt werden, während gewöhnlich die unteren Grenzwerte U bzw. die zusätzlichen Werte $U_1$ und $U_2$ als Hinweise für besonders gute Qualität genutzt werden, die entweder einen Eingriff in die Produktion ratsam erscheinen lassen oder eine Verwertung entsprechend gefertigter, besonders guter Produkte für Sonderverwendungen ermöglichen.

Das hier vorgeschlagene Verfahren zur Herstellung und/oder Verarbeitung eines bahnförmigen Materials, insbesondere von Papier, mit dem mindestens einen bevorzugt traversierenden Messsystem umfasst ganz besonders bevorzugt als zusätzlichen Veredelungsprozess das mindestens einmalige Beschichten des bahnförmigen Materials mit einer Masse, wobei die Masse ausgesucht ist aus der Gruppe, umfassend: Druckfarbe, Streichfarbe, Lack, Extrudermasse.

Das hier vorgeschlagene Verfahren zur Herstellung und/oder Verarbeitung eines bahnförmigen Materials, insbesondere von Papier findet ganz besonders bevorzugt auf mindestens zwei unabhängigen Maschinen statt. Die erste Maschine dient dann der Herstellung des bahnförmigen Materials, während die zweite Maschine zur Veredelung des zuvor erstellten bahnförmigen Materials dient. In einem solchen ganz besonders bevorzugten Fall ist diese zweite Maschine ausgesucht aus der Gruppe, umfassend: Druckmaschine, Streichmaschine, Lackiermaschine, Extrudiermaschine. Eine solche zweite Maschine kann innerhalb der gleichen Firma wie die erste Maschine betrieben werden, sie kann aber auch in einer Kundenfirma oder in der Firma eines beliebigen Lizenznehmers betrieben werden. Das erfindungswesentliche Messsystem kann dabei innerhalb der ersten Maschine und/oder innerhalb der zweiten Maschine zum Einsatz kommen.

Als Messsystem bieten sich insbesondere

- Systeme zur Bestimmung der Dicke,

- Systeme zur Bestimmung der flächenbezogenen Masse des gesamten bahnförmigen Materials und/oder seiner Beschichtung und

- Systeme zur Bestimmung der relativen und/oder der absoluten Feuchte

an, ohne darauf jedoch im Sinne der vorliegenden Erfindung beschränkt zu sein.

Die vorliegende Erfindung soll anhand eines Beispiels weitergehend erläutert werden.

In einem Auflöser wird zur Ausbildung einer Pulpe eine Mischung aus gebleichtem Nadelholzsulfatzellstoff und Eukalyptuszellstoff zusammen mit Wasser vorgelegt, bis ein Feststoffgehalt hier von 4,2 % erreicht ist. Die Pulpe enthält als weitere Zuschlagstoffe beispielsweise Pigmente in üblichen Mengen und Natronlauge. Die fertig gestellte Pulpe wird einer Mahlung unterzogen und dann mit weiteren Zuschlagstoffe wie beispielsweise Leimungsmitteln einer Papiermaschine zugeführt, die aus dieser Pulpe bei einer Bahngeschwindigkeit hier von 1050 m/sec eine Rohpapierbahn mit einer flächenbezogenen Masse von 42 g/m$^2$ erstellt. Die Einstellungen für die Papiermaschine werden moderat in verschiedene Richtungen verändert und dann jeweils notiert, um zum einen besonders gleichmäßiges Rohpapier und zum anderen ungleichmäßiges, hier streifiges Rohpapier zu erhalten.

Unter Nutzung eines traversierenden Messsystems wird an n = 512 Messpositionen quer über die 5,9 Meter breite Rohpapierbahn die jeweilige flächenbezogene Masse (otro) bestimmt. Bei einem Zeitintervall At = 60 Minuten, innerhalb dessen ein Tambour mit fertig gestelltem Rohpapier aufgewickelt wird, kommt es zu 60 Traversierungen des Messsystems, was bedeutet, dass pro Messposition m = 60 verschiedene Einzelmesswerte aufgenommen werden. Zahlreiche Proben zum einen mit besonders gleichmäßigem Rohpapier und zum anderen mit auffallend ungleichmäßigem, streifigem Rohpapier aus dem Tambour mit der fertig gestellten Rohpapierbahn werden ein erstes Mal visuell von mehreren Produktionsverantwortlichen im Durch- und im Streiflicht begutachtet und in Relation gesetzt zu Querprofilen, die für jede einzeln betrachtete Papiermaschineneinstellung gebildet sind aus der jeweiligen Gesamtheit aller Mittelwerte $\bar{y}_i$ für die 512 Messpositionen. Bereits zu diesem frühen Zeitpunkt zeigt sich, dass eine nach

## Formel 10:

$$Q = \frac{\sum_{i=1}^{n} |M_i|}{\sum_{i=1}^{n} \frac{\bar{y}_i}{n}}$$

bestimmte Qualitätszahl Q = 2,7 bei der hier genutzten Papiermaschine für das hier betrachtete Rohpapier für eine gute und hervorragend weiterzuverarbeitende Qualität des Rohpapiers steht, während eine Qualitätszahl Q = 4,5 bei der hier genutzten Papiermaschine für das hier betrachtete Rohpapier für eine schlechte Qualität des Rohpapiers steht. Für beide nach Formel 10 bestimmte Qualitätszahlen Q = 2,7 und Q = 4,5 geben die Figuren 1 und 2 die aufgenommenen Querprofile aus der jeweiligen Gesamtheit aller Mittelwerte $\bar{y}_i$ für die n = 512 Messpositionen bei m = 60 Traversierungen wieder.

Mittels zweier Schlitzgießer-Auftragwerke (Curtain Coater) werden innerhalb ein und desgleichen Durchgangs durch eine Streichmaschine, die als eigenständige Maschine separat von der oben genannten Papiermaschine genutzt wird, auf die Bahn sowohl des gleichmäßigen wie auch des streifigen Rohpapiers zunächst eine die Rohpapierbahn vollständig abdeckende Zwischenschicht von 6 g/m$^2$ und anschließend eine wärmeempfindliche Aufzeichnungsschicht mit einer flächenbezogenen Masse von 2,5 g/m$^2$ aufgetragen. Dabei kommt zur Ausbildung der Zwischenschicht eine Rezeptur zum Einsatz, die als wesentliche Komponenten anorganisches Pigment (80 Gew.-%) und Latex-Bindemittel (20 Gew.-%) enthält. Zur Ausbildung der wärmeempfindlichen Aufzeichnungsschicht kommt eine Rezeptur zum Einsatz, deren wesentliche Komponenten in der folgenden Tabelle 1 wiedergegeben sind:

Tabelle 1:

| Bestandteil | Gew.-% (otro) |
|---|---|
| Farbbildner | 9,5 |
| Farbakzeptor | 21,5 |
| Sensibilisator | 21,5 |
| Pigment | 25,0 |
| Bindemittel | 17,0 |

Nach der Trocknung von Zwischenschicht und wärmeempfindlicher Aufzeichnungsschicht mittels mehrerer Schwebetrockner innerhalb der Streichmaschine werden die derart gestrichenen Papierbahnen zum einen mit dem besonders gleichmäßigen Rohpapier und zum anderen mit dem besonders ungleichmäßigen, hier streifigen Rohpapier ein zweites Mal visuell im Durch- und im Streiflicht und zusätzlich im aufgerollten Zustand auf einem Tambour taktil durch Abfühlen der Rollenoberfläche von mehreren Produktionsverantwortlichen begutachtet. Während die gestrichene Papierbahn mit dem besonders gleichmäßigen Rohpapier als Beispiel für gute Ware angesehen wird, kann die gestrichene Papierbahn mit dem streifigen Rohpapier als Beispiel für mangelhafte Ware angesehen werden. Die zu dem gestrichene Papierbahn mit dem besonders gleichmäßigen Rohpapier gehörige Qualitätszahl wird dann entsprechend als Ziel-Qualitätszahl Q = 2,7 festgesetzt, der obere Wert O ergibt sich in diesem Fall aus den Qualitätszahlen für die gestrichene Papierbahn mit dem streifigen Rohpapier mit O = 4,2. Der untere Wert für ganz besonders gute Rohpapiere kann hier zunächst als Schätzwert festgesetzt werden mit U = 2,2.

Im Rahmen nachfolgender Produktionen und unter Berücksichtigung von Kundenreaktionen und/oder Kundenreklamationen können die Werte von U, Q und O immer wieder angepasst werden.

**Patentansprüche**

1. Verfahren zur Herstellung und/oder Verarbeitung eines bahnförmigen Materials, insbesondere von Papier, mit mindestens einem Messsystem, das innerhalb eines festen Zeitintervalls $\Delta t$ über mindestens einen Teil der Bahnbreite s des bahnförmigen Materials an n verschiedenen Messpositionen $x_{i\,(i=1,..,n)}$ pro Messposition jeweils m Messwerte $y_{ik(i=1,...,n;k=1,...,m)}$ aufnimmt,
**dadurch gekennzeichnet, dass**
das Verfahren die Bestimmung einer rationalen Qualitätszahl Q umfasst, deren Wert für eine als akzeptabel angesehene Qualität des bahnförmigen Materials innerhalb eines für den Herstellungsprozess jeweils empirisch festzulegenden Akzeptanzbereichs mit einer unteren Grenze U und einer oberen Grenze O liegt,
wobei U und O jeweils rationale Zahlen sind mit der Bedingung gemäß:

## Formel 1:

$$U \leq Q \leq O,$$

wobei sich der Wert der Qualitätszahl Q als Funktion der Momente $M_i$ berechnet gemäß Formel 2 mit:

## Formel 2:

$$Q = f(M_i)$$

und wobei sich die Momente $M_i$ berechnen aus dem Funktionswert der kubischen Spline-Funktion S(x) für die n verschiedenen Messpositionen gemäß Formel 3 mit:

## Formel 3:

$$S(x) = \alpha_i + \beta_i \cdot (x - x_i) + \gamma_i \cdot (x - x_i)^2 + \delta_i \cdot (x - x_i)^3$$

8

mit:

x ist Laufvariable in Richtung der Bahnbreite s des bahnförmigen Materials im Intervall $[x_i, x_{i+1}]$

$X_{i\,(i=1,..,n)}$ sind die Messpositionen, mathematisch auch als Stützstellen der kubischen Spline-Funktion bezeichnet, für die Momente $M_i$,

und wobei für die Momente $M_i$, ausgehend von den zugrundeliegenden Messwerten $y_{ik}$ und unter Nutzung vorhergehender Formel 3 als Darstellung des Funktionswertes der kubischen Spline-Funktion $S(x)$, die Formel 4 gilt mit:

### Formel 4:

$$M_i = 2 \cdot \gamma_{i\,(i=1,...,n-1)}, \quad M_n = 2 \cdot \gamma_{n-1} + \delta_{n-1} \cdot (x_n - x_{n-1})$$

2. Verfahren nach Patentanspruch 1, **dadurch gekennzeichnet, dass** das Messsystem als ein traversierendes Messsystem ausgebildet ist.

3. Verfahren nach einem der Patentansprüche 1 oder 2, **dadurch gekennzeichnet, dass** die Funktion zur Berechnung der Qualitätszahl Q aus den Momenten $M_i$ ausgesucht ist aus der Liste, umfassend:

- Summe der Absolutwerte der Momente $M_i$ (Betragssummennorm)
- Maximumwert der Absolutwerte aller Momente $M_i$ (Maximumsnorm)
- Wurzel aus der Summe der Quadrate der Momente $M_i$ (Euklidische Norm)
- Varianz aus den Momenten $M_i$
- $\sigma$-Regeln beliebiger Stufe aus den Momente $M_i$ ($\sigma$-Wert, 2-$\sigma$-Wert, 3-$\sigma$-Wert, ...)
- Mittelwert aus den absoluten Momenten $M_i$

4. Verfahren nach Patentanspruch 3, **dadurch gekennzeichnet, dass** die Funktion zur Berechnung der Qualitätszahl Q aus den Momenten $M_i$ die Summe der Absolutwerte der Momente $M_i$ ist geteilt durch den Mittelwert gemäß Formel 10, mit:

### Formel 10:

$$Q = \frac{\sum_{i=1}^{n} |M_i|}{\sum_{i=1}^{n} \frac{\bar{y}_i}{n}}$$

5. Verfahren nach einem der Patentansprüche 1 bis 4, **dadurch gekennzeichnet, dass** Verfahren einen zusätzlichen Veredelungsprozess umfasst, der unterbunden wird, wenn die Qualitätszahl Q für das zur Veredelung vorgesehene bahnförmige Material außerhalb des Akzeptanzbereichs mit der unteren Grenze U und der oberen Grenze O liegt.

6. Verfahren nach Patentanspruch 5, **dadurch gekennzeichnet, dass** der zusätzliche Veredelungsprozess das mindestens einmalige Beschichten des bahnförmigen Materials mit einer Masse umfasst, wobei die Masse ausgesucht ist aus der Gruppe, umfassend: Druckfarbe, Streichfarbe, Lack, Extrudermasse.

7. Verfahren nach einem der Patentansprüche 5 oder 6, **dadurch gekennzeichnet, dass** das Verfahren auf mindestens zwei unabhängigen Maschinen stattfindet, von denen die erste Maschine zur Herstellung des bahnförmigen Materials und die zweite Maschine zur Veredelung des zuvor erstellten bahnförmigen Materials dient.

8. Verfahren nach einem der Patentansprüche 1 bis 7, **dadurch gekennzeichnet, dass** das mindestens eine Messsystem ein System zur Bestimmung der Dicke ist.

9. Verfahren nach einem der Patentansprüche 1 bis 7, **dadurch gekennzeichnet, dass** das mindestens eine Messsystem ein System zur Bestimmung der flächenbezogenen Masse ist.

**10.** Verfahren nach einem der Patentansprüche 1 bis 7, **dadurch gekennzeichnet, dass** das mindestens eine Messsystem ein System zur Bestimmung der Feuchte ist.

**Claims**

**1.** Method of producing and/or processing a web-form material, especially paper, with at least one measuring system which, within a fixed time interval $\Delta t$, records at n different measurement positions $x_{i(i = 1,..., n)}$ across at least a portion of the web width s of the web-form material m measured values $y_{ik(i=1,...,n; k=1,..., m)}$ per measurement position, **characterised in that**
the method comprises the determination of a rational quality number Q, the value of which for a quality of the web-form material that is regarded as acceptable lies within an acceptance range having a lower boundary U and an upper boundary O, which range is to be ascertained empirically for each production process,
wherein U and O are each rational numbers subject to the condition in accordance with:

## formula 1:

$$U \leq Q \leq O,$$

wherein the value of the quality number Q is calculated as a function of the moments $M_i$ in accordance with formula 2:

## formula 2:

$$Q = f(M_i)$$

and wherein the moments $M_i$ are calculated on the basis of the function value of the cubic spline function S(x) for the n different measurement positions in accordance with formula 3:

## formula 3:

$$S(x) = \alpha_i + \beta_i \bullet (x - x_i) + \gamma_i \bullet (x - x_i)^2 + \delta_i \bullet (x - x_i)^3$$

where:

x is an indexed variable in the direction of the web width s of the web-form material at intervals $[x_i, x_{i+1}]$
$x_{i\,(i = 1,..., n)}$ are the measurement positions, mathematically also termed supporting points of the cubic spline function, for the moments $M_i$,

and wherein for the moments $M_i$, on the basis of the underlying measured values $y_{ik}$ and utilising the above formula 3 to represent the function value of the cubic spline function S(x), the formula 4 applies:

## formula 4:

$$M_i = 2 \bullet \gamma_{i\,(i = 1,.., n-1)}, \quad M_n = 2 \bullet \gamma_{n-1} + \delta_{n-1} \bullet (x_n - x_{n-1})$$

.

**2.** Method according to patent claim 1, **characterised in that** the measuring system is in the form of a traversing measuring system.

**3.** Method according to either one of patent claims 1 and 2, **characterised in that** the function for calculating the quality number Q on the basis of the moments $M_i$ is selected from the list comprising:

• sum of the absolute values of the moments $M_i$ (absolute value sum norm)
• maximum value of the absolute values of all moments $M_i$ (maximum norm)
• root of the sum of the squares of the moments $M_i$ (Euclidian norm)
• variance based on the moments $M_i$
• $\sigma$ rules of any stage based on the moments $M_i$ ($\sigma$-value, 2-$\sigma$-value, 3-$\sigma$-value, ...)
• mean value based on the absolute moments $M_i$.

**4.** Method according to patent claim 3, **characterised in that** the function for calculating the quality number Q on the basis of the moments $M_i$ is the sum of the absolute values of the moments Mi divided by the mean value in accordance with formula 10:

<u>formula 10:</u>

$$Q = \frac{\sum\limits_{i=1}^{n} |M_i|}{\sum\limits_{i=1}^{n} \dfrac{\bar{y}_i}{n}} \; .$$

**5.** Method according to any one of patent claims 1 to 4, **characterised in that** the method comprises an additional finishing process which is inhibited when the quality number Q for the web-form material intended for finishing lies outside the acceptance range with the lower boundary U and the upper boundary O.

**6.** Method according to patent claim 5, **characterised in that** the additional finishing process comprises the coating, at least once, of the web-form material with a composition, the composition being selected from the group comprising: printing ink, coating colour, lacquer, extruder composition.

**7.** Method according to either one of patent claims 5 and 6, **characterised in that** the method takes place on at least two independent machines, of which the first machine serves for producing the web-form material and the second machine serves for finishing the web-form material previously created.

**8.** Method according to any one of patent claims 1 to 7, **characterised in that** the at least one measuring system is a system for determining the thickness.

**9.** Method according to any one of patent claims 1 to 7, **characterised in that** the at least one measuring system is a system for determining the mass per unit area.

**10.** Method according to any one of patent claims 1 to 7, **characterised in that** the at least one measuring system is a system for determining the moisture content.

**Revendications**

**1.** Procédé pour la fabrication et/ou la transformation d'un matériau en feuille continue, en particulier de papier, avec au moins un système de mesure, qui à l'intérieur d'un intervalle de temps fixe $\Delta t$, enregistre sur au moins une partie de la largeur s du matériau en feuille continue, en chacune de n positions de mesure différentes $x_{i(i = 1,...n)}$, et par position de mesure, m valeurs de mesure $Y_{ik(i=1,...,n\,;\,k=1,...m)}$,
**caractérisé en ce que** le procédé comprend la détermination d'un indice de qualité Q rationnel, dont la valeur, pour une qualité considérée comme acceptable du matériau en feuille continue, est située à l'intérieur d'un domaine d'acceptation, devant être déterminé d'une manière empirique pour chaque opération de fabrication, ayant une limite inférieure U et une limite supérieure O, où U et O sont chacun des nombres rationnels, avec les conditions suivantes :

<u>Formule 1 :</u>

$$U \le Q \le O$$

la valeur de l'indice de qualité Q étant calculée en fonction des moments $M_i$ selon la Formule 2 :

$$\underline{\text{Formule 2 :}}$$

$$Q = f(M_i)$$

et les moments Mi étant calculés à partir de la valeur de la fonction spline cubique S(x) pour les n différentes positions de mesure selon la Formule 3 :

$$\underline{\text{Formule 3 :}}$$

$$S(x) = \alpha_i + \beta_i \cdot (x - x_i) + \gamma_i \cdot (x - x_i)^2 + \delta_i \cdot (x - x_i)^3$$

où :

x est une grandeur réglée dans la direction de la largeur s du matériau en feuille continue dans l'intervalle [$x_i$ ,$x_{i+1}$], $x_{i(i = 1..., n)}$ représente les positions de mesure, appelées aussi d'un point de vue mathématique les points d'appui de la fonction spline cubique, pour les moments $M_i$, et, pour les moments $M_i$, en partant des valeurs de mesure $y_{ik}$ à utiliser, et en faisant appel à la Formule 3 ci-dessus, en tant que représentation de la valeur de la fonction spline cubique S(x), on a la Formule 4 :

$$\underline{\text{Formule 4 :}}$$

$$M_i = 2 \cdot \gamma_{i \, (i = 1,..., n-1)}, \; M_n = 2 \cdot \gamma_{n-1} + \delta_{n-1} \cdot (x_n - x_{n-1})$$

**2.** Procédé selon la revendication 1, **caractérisé en ce que** le système de mesure est conçu comme un système de mesure transversal.

**3.** Procédé selon l'une des revendications 1 ou 2, **caractérisé en ce que** la fonction permettant le calcul de l'indice de qualité Q à partir des moments $M_i$ est choisie dans la liste comprenant :

- la somme des valeurs absolues des moments $M_i$ (norme de la somme des modules)
- la valeur maximale des valeurs absolues de la totalité des moments $M_i$ (norme des maxima)
- la racine de la somme des carrés des moments $M_i$ (norme euclidienne)
- la variance obtenue à partir des moments $M_i$,
- les règles $\sigma$ de degré quelconque à partir des moments $M_i$ (valeur $\sigma$, valeur 2-$\sigma$, valeur 3-$\sigma$, ...)
- la moyenne des moments absolus $M_i$.

**4.** Procédé selon la revendication 3, **caractérisé en ce que** la fonction permettant le calcul de l'indice de qualité Q à partir des moments $M_i$ est la somme des valeurs absolues des moments $M_i$, divisée par la valeur moyenne selon la Formule 10 :

$$\underline{\text{Formule 10 :}}$$

$$Q = \left. \sum_{i=1}^{n} |M_i| \middle/ \sum_{i=1}^{n} \frac{\bar{y}_i}{n} \right.$$

**5.** Procédé selon l'une des revendications 1 à 4, **caractérisé en ce que** le procédé comprend un processus supplémentaire d'ennoblissement qui est empêché lorsque l'indice de qualité Q, pour le matériau en feuille continue prévu pour l'ennoblissement, se trouve à l'extérieur du domaine d'acceptation ayant la limite inférieure U et la limite supérieure O.

**6.** Procédé selon la revendication 5, **caractérisé en ce que** le processus supplémentaire d'ennoblissement comprend l'application, au moins une fois, d'une masse sur le matériau en feuille continue, la masse étant choisie dans le groupe comprenant une encre d'imprimerie, une sauce de couchage, un vernis, une masse pour extrusion.

**7.** Procédé selon l'une des revendications 5 ou 6, **caractérisé en ce que** le procédé est mis en oeuvre sur au moins deux machines indépendantes, la première machine servant à la fabrication du matériau en feuille continue, et la deuxième machine servant à l'ennoblissement du matériau en feuille continue fabriqué au préalable.

**8.** Procédé selon l'une des revendications 1 à 7, **caractérisé en ce que** le au moins un système de mesure est un système destiné à la détermination de l'épaisseur.

**9.** Procédé selon l'une des revendications 1 à 7, **caractérisé en ce que** le au moins un système de mesure est un système destiné à déterminer la masse surfacique.

**10.** Procédé selon l'une des revendications 1 à 7, **caractérisé en ce que** le au moins un système de mesure est un système destiné à la détermination de l'humidité.

Figur 1:

Querprofil aus der jeweiligen Gesamtheit aller Mittelwerte $\overline{y}_i$ für die n = 512 Messpositionen bei m = 60 Traversierungen mit Q = 4,5, das

bedeutet, außerhalb des empirisch festgelegten Akzeptanzbereichs mit U = 2,2 ≤ Q ≤ O = 4,2.

Figur 2:

Querprofil aus der jeweiligen Gesamtheit aller Mittelwerte $\bar{y}_i$ für die n = 512 Messpositionen bei m = 60 Traversierungen mit Q = 2,7, das

bedeutet, innerhalb des empirisch festgelegten Akzeptanzbereichs mit U = 2,2 ≤ Q ≤ O = 4,2.

EP 2 412 869 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1473407 A1
- WO 03072874 A1

- WO 2010080869 A1